# EUROPEAN PATENT SPECIFICATION

(11) **EP 2 114 391 B1**
(45) Date of publication and mention of the grant of the patent: **16.09.2020**
(21) Application number: 08705167.8
(22) Date of filing: 22.01.2008
(51) Int. Cl.: A61K 31/19, A61K 9/20, A61K 9/16, A61P 1/04, A61P 35/00

(54) **A PREPARATION CONTAINING SODIUM BUTYRATE AND THE APPLICATION OF A PREPARATION CONTAINING SODIUM BUTYRATE**
NATRIUMBUTYRAT ENTHALTENDES ZUBEREITUNG UND APPLIKATION EINER NATRIUMBUTYRAT ENTHALTENDEN ZUBEREITUNG
PREPARATION CONTENANT DU BUTYRATE DE SODIUM ET L' APPLICATION D'UNE PREPARATION CONTENANT DU BUTYRATE DE SODIUM

(30) Priority: 25.01.2007 PL 38160307
(43) Date of publication of application: 11.11.2009
(73) Proprietor: Biolek SP. z o.o., 01-231 Warszawa (PL)
(72) Inventor: BORYCKA, Katarzyna, PL-01-026 Warszawa (PL); KICIAK, Adam, PL-01-026 Warszawa (PL); KOTUNIA, Anna, Maria, PL-01-926 Warszawa (PL); MICHALOWSKI, Pawel, Andrzej, PL-05-126 Nieporet (PL); VALVERDE PIEDRA, Jose, Luis, PL-21-003 Ciecierzyn (PL); ZABIELSKI, Romuald, PL-02-997 Warszawa (PL)
(74) Representative: Witek, Rafal
(86) International application number: PCT/PL2008/000008
(87) International publication number: WO 2008/091170

(56) References cited:
- EP-A2- 2 025 243
- WO-A1-95/13801
- US-A1- 2004 009 206
- VERNIA P ET AL: "Combined oral sodium butyrate and mesalazine treatment compared to oral mesalazine alone in ulcerative colitis: Randomized, double-blind, placebo-controlled pilot study" DIGESTIVE DISEASES AND SCIENCES, vol. 45, no. 5, May 2000 (2000-05), pages 976-981, XP002477128 ISSN: 0163-2116
- A. Di Sabatino ET AL: "Oral butyrate for mildly to moderately active Crohn's disease", ALIMENTARY PHARMACOLOGY & THERAPEUTICS., vol. 22, no. 9, 1 November 2005 (2005-11-01), pages 789-794, XP055357191, GB ISSN: 0269-2813, DOI: 10.1111/j.1365-2036.2005.02639.x
- BANASIEWICZ ET AL: "Microencapsulated butyrate reduces the frequency of abdominal pain in patients with irritable bowel syndrome", COLORECTAL DISEASE, vol. 15, no. 2, 27 June 2012 (2012-06-27), pages 204-209,
- TARNOWSKI ET AL: "Outcome of treatment with butyric acid in irritable bowel syndrome -preliminary report", GASTROENTEROLOGIA PRAKTYCZNA, vol. 1, 1 January 2011 (2011-01-01), pages 1-6,
- BANASIEWICZ: "Quality of life and the clinical symtoms at the patients with irritable bowel syndrome treated complementary with protected sodium butyrate", GASTROENTEROLOGIA PRAKTYCZNA, vol. 5, 1 January 2011 (2011-01-01), pages 1-9,
- KARAKULSKA ET AL: "Butiric acid in inhibition of diarrhoea in course of chemotherapy-case report", ONKOL PRAK KLIN, vol. 7, no. 1, 1 January 2011 (2011-01-01) , pages 40-42,
- KROKOWICZ ET AL: "Microencapsulated sodium butyrate administered to patients with diverticulosis decreases incidence of diverticulitis-a prospective randomized study", INT J COLORECTAL DIS, 18 December 2013 (2013-12-18), pages 387-393,

## Description

The subject of the present invention is a preparation containing butyric acid or its pharmaceutically permissible salt, particularly sodium butyrate, in a triglyceride matrix of a specially selected composition and application of said preparation in the treatment of gastrointestinal diseases in humans.

A preparation, according to the present invention, is designed to exert a positive effect on intestinal epithelium cells and other cell populations in the intestinal wall through stimulating their growth and/or maturation, and to inhibit the proliferation of tumour cells, which is meant to result in the reconstruction of disease-altered intestinal walls in the human. Due to its properties, the preparation may be used in humans as a drug for the treatment of acute and chronic intestinal inflammations of varying aetiology and in colorectal cancer therapy.

Butyric acid is a product of bacterial fermentation of carbohydrates in the rumen, the large intestine of omnivores (i.e. *Domestic pig*) and the human colon. It belongs to the SCFA group (short-chain fatty acids), which are the chief source of anions in the colon of humans and mammals. Bacteria which produce butyric acid mainly include sugar fermenters such as *Clostridium spp., Eubacterium spp., Fusobacterium spp., Butyrivibrio spp.* It is selectively transported from the colonic lumen through the cell membrane into colonocytes through the pH-gated, electrically neutral anion exchanger (Ritzhaupt et al., 1998), via both active and passive pathway (Charney 1998, Hadjiagapiou et al. 2000). Sodium butyrate is the sodium salt of butyric acid characterized by a solid state, and a greater particle stability than that of butyric acid. In an aqueous solution sodium butyrate easily dissociates into butyric acid.

*In vitro*, sodium butyrate induces the differentiation of brush-border enterocytes, changes in the surface of microvilli and the secretion of mucin. It influences water and sodium reabsorption (Holtug et al., 1992; Binder et al., 1989) and is a significant energy source for colonocytes (Schappeach et al., 1992; Firmansyah et al., 1989; Reodriger, 1980). In *in vivo* studies in juvenile pigs, it elicits a strong trophic effect on the mucous membrane of the small intestine following *oral* application (Kotunia et al., 2004). It increases local blood flow and stimulates gastrointestinal tract motility. It also exerts a cytoprotective and anti-inflammatory effect on gastric and intestinal mucous membrane cells (Andoh et al.,1999; Pander et al., 2000; Chapman et al., 1994) and stimulates the elimination and growth inhibition of tumour cells (McIntyre et al., 1993; Aviv-Green et al., 2002; Velazquez et al.,1996; Hassing et al.,1997; Witehead et al., 1986).

In clinical practice, butyrate is sporadically used in patients with short bowel syndrome following extensive resections of the small intestine (due to ischemia or inflammation), during long-term total parenteral nutrition or enteral nutrition as well as following radiotherapy in order to stimulate adaptive processes in the intestinal epithelium (Jeppesen et al. 2002, Thompson et al. 1996). Despite the limited number of randomized studies evaluating the effects of SCFA on non-specific inflammatory bowel disease, a significant reduction of disease symptoms has been observed many times (Kanauchi et al., 2001; Kanauchi et al., 2002) as well as a decrease in the intensity of the inflammation in the intestine as a result of the administration of butyrate, both in Crohn's disease as well as in ulcerative colitis (Galvez et al., 2005). Butyrate has also been used successfully in the treatment of non-specific bowel inflammation following proctocolectomy with ileo-anal anastomosis (Welters et al., 2002) and in the treatment of *diversion colitis.* In the latter case, the effects of *diversion colitis* receded following SCFA lavage (Mortensen et al., 1991; Guillemot et al., 1991; Eggenberger et al., 2001).

The effect of SCFA on the composition of intestinal microflora is significant in the maintenance of proper intestinal structure, functionality and integrity. A healthy mucosa and probiotic microflora constitute an effective barrier against bacterial pathogens. The presence of SCFA-producing bacteria inhibits the growth of such species as *Escherichia coli*, *Campylobacter* and *Salmonella* (Chen et al., 2005), both via competition as well as the secretion of antibacterial molecules and immunostimulation. The antibacterial activity of SCFA (acetate, propionate, n-butyrate; 60:30:40 mM) in a study on rabbits infected with *Shigella* (Rabbani et al., 1999). Disruptions of the composition of intestinal microflora play a significant role in the pathogenesis of diarrhoeas occurring during antibiotic therapy (Young and Schmidt, 2004). A close correlation has been observed between the occurrence of antibiotic-related diarrhoea and a decrease of butyrate-producing bacteria in the intestinal lumen (Bartlett, 2002), which suggests that it plays a significant role in intestinal functioning.

One of the many physiological functions of SCFA is the stimulation of reabsorption of sodium in the colon. In animal studies, it was shown that SCFA activity in this respect consists of stimulating the expression and activity the Na⁺/H⁻ ion pump in colonocytes. In animal studies using cholera toxin, it was shown that butyrate is very effective in limiting the colonic secretion of water, sodium, chloride and potassium (Rabbani et al., 1999). This has been confirmed clinically in cholera patients, in whom the administration of dietary fibre which increase colonic SCFA greatly reduced bowel water and electrolyte loss, by the same token easing the disease symptoms (Ramakrishna et al., 2000). SCFA also play a beneficial role in bacterial and viral diarrhoeas, through stimulating epithelial immune mechanisms via Toll-like receptor activation, as well as through the production of Interleukin-10 (I1-10) and interferon γ (IFNγ) and the stimulation of immunoglobulin A (IgA) production (Roller et al., 2004; Zapolska-Downar et al., 2004). It was shown in numerous studies that diarrhoea resulting from long-term intra-intestinal feeding is a consequence of atrophic changes in intestinal mucosa and disruptions of intestinal microflora resulting from deficient levels of SCFA (Schneider et al., 2005; Schneider et al., 2000). Similar observations were obtained from the clinical observation of children chronically treated intra-intestinally during Crohn's disease (Lionetti et al., 2005).

Fatty acids, including butyric acid, are a chief source of energy for cells of the colonic mucosa (Reodriger, 1980), and of the greatest importance to colonocytes in the distal region of the colon. Theoretically, butyrate may constitute 80% of the energy needed by colonocytes and some 5-10% of the overall systemic energetic requirements. The strong trophic effect of butyric acid on the mucous membrane of the small intestine has been observed in experimental animals (Kotunia et al., 2004, Guilloteau et al., 2004). A decrease in the intestinal butyric acid concentration leads to the atrophy of colonic mucosa, which is usually explained by a decrease in the availability of substrates to colonocytes (Sakata, 1987; Janne et al., 1977). On the other hand, administration of butyrate into the colon lumen induces weight gain, an increase in DNA synthesis and the depth of intestinal crypts (Kripke et al., 1989). In rats, *in vivo*, both intravenous and intracolonic SCFA infusions significantly reduced the atrophy of mucosa caused by long-term total parenteral nutrition (TPN) (Koruda et al., 1990). The effectiveness of SCFA as a strong factor for stimulating the reconstruction of intestinal mucosa was also shown clinically in patients following extensive resection of the bowel (Jeppesen et al., 2002; Thompson et al., 1996).

A high concentration of butyric acid achieved through the fermentation of insoluble dietary fibre or following the anal administration of butyrate may inhibit early and late stages of colon oncogenesis through the regulation of transcription, expression and activation of key proteins of the apoptotic cascade (Aviv-Green et al., 2002). The mechanism of oncogenesis inhibition by butyric acid is explained by its influence on the cell differentiation, the cell cycle and apoptosis (Aviv-Green et al., 2000). In *in vitro* studies on tumour cell lines it was shown that butyrate exerts an inhibitory effect, at doses not toxic to the cells (1 mmolM/1), on DNA synthesis, with a parallel weak inhibition of RNA and protein synthesis, and by arresting cells in the G1 stage of the cell cycle (Withehead et al., 1986). The effect of butyrate on cell proliferation in the colon of patients at risk of developing colon cancer was examined. In colonic epithelial cells of a pre-tumour proliferation type (the proliferation zone moved to the upper 40% of the colonic crypt) a significant decrease in the proliferation of upper crypt cells was observed, along with increased proliferation in the lower portion of the crypt (60%), meaning the physiological proliferation zone of these cells (Scheepach et al., 1992).

The growth, differentiation, migration, adhesion and metastasizing ability of tumour cells are dependent on the activity of laminins and basal membrane glycoproteins. The expression of laminin receptors varies inversely to the degree of differentiation and proportionately to the metastasizing potential of a tumour. Butyrate stimulates the differentiation of colon tumour cells (HT-29) likely through limiting their adhesion to laminin (Wilson et al., 1992), thus influencing tumour growth and differentiation. Pander et al. (2000) showed that butyrate limits the ability of tumours to metastasize influencing the synthesis of stromelisin-1, a metalloprotease (MMP) responsible for the degradation of proteins in the connective tissue of vascular walls. Damage to the vascular wall facilitates the migration of tumour cells and leads to the formation of metastases. Various MMPs play a role in the development of colon cancer and non-oncogenic diseases such as Crohn's disease and ulcerative colitis (Kolomecki, 2000).

Chapman et al. (1994) showed that inflamed colonic mucosa captures much more butyrate than glutamine or glucose. This confirms the hypothesis of Reodriger (1980), that ulcers of the colon are a disease connected to energy deficiencies. CO₂ production from butyrate amounts to 72 pmol/h in 1 µg dry mass of healthy colonocytes. In patients with colitis this falls to 36 pmol/h in 1 µg dry mass. Butyrate oxidation is also reduced in mucosa from 472 to 272 pmol/h in 1 µg of mucosal protein (Chapman et al., 1994). The exogenous administration of butyrate, leading to its superphysiological concentrations, may aid in deficient butyrate oxidation.

Experiments showed that the application of butyrate perfusions causes a significant reduction of inflammation, and a decrease in the extent of ulceration of the colon wall in the rat (Andoh et al., 1999). The effectiveness of butyrate enemas is confirmed by clinical observations in patients with ulcerative colitis (Han et al., 1999; Scheppach et al., 1997). The effective range of enemas, however, is limited to within 50-60 cm of the anus. In patients with colon inflammations and strong diarrhoea, who have very low SCFA concentrations, an improvement in the state of the colonic mucous membrane was observed in clinical tests, as well as using endoscopy and histological preparations following butyrate enemas. An anal butyrate perfusion in patients with ulcerative colitis yielded positive effects due to the anti-inflammatory activity of butyrate on target intestinal epithelium cells (Böcker et al., 2003). In *in vitro* studies, it was shown that butyrate, is capable of reducing the inflammation of the intestinal epithelium in a dose dependent manner, through limiting the TNF-α stimulated secretion of 11-8. It also inhibits the activation of TNF-α itself, the main and strongest-acting inflammatory cytokine (Akira et al., 1999). The direct anti-inflammatory activity of butyrate is connected with the inhibition of the migration of the KappaB nuclear factor (NPκB) and its binding of DNA, and by the same token the inhibition of transcription and production of pro-inflammatory cytokines (Segain et al., 2000). SCFA are also able to inhibit lymphocyte activity and proliferation (Chapman et al., 2001) and to inhibit neutrophil myeloperoxydase activity responsible for the degradation of the inflamed intestinal wall (Liu et al., 2001).

An alternative method of delivering SCFA into the bowel with inflammation is to supply substrates for their production in the diet, dietary fibre. Mitsuyama et al. (1998) demonstrated the effectiveness of this approach in a pilot study in patients with ulcerated colon inflammation. Over a 4-week observation, they noted an improvement in the patients' clinical state and the endoscopic results, with a good toleration of the preparation and no undesirable effects. Similar results were obtained by Welters et al. (2002) in patients with recurrent pouchitis following extensive resection of the large intestine with ileo-anal anastomosis and ileal pouch-anal anastomosis (J-pouch). The pouchitis, characterized by chronic inflammation of the mucosa was significantly reduced after a 3-week supplementation period with inuline, increasing the colonic SCFA concentration.

In the study presented in "Combined oral sodium butyrate and mesalazine treatment compared to oral mesalazine alone in ulcerative colitis: randomized, double-blind, placebo-controlled pilot study" (Vernia et al., Dig Dis Sci. 2000 May; 45(5):976-8) the authors shown data suggesting that oral butyrate may improve the efficacy of oral mesalazine in active ulcerative colitis in form oral dosage of sodium butyrate tablets, coated with a pH-dependent soluble polimer, wherein oral butyrate is safe and well tolerated.

In summary it should be stated that butyrate is the SCFA, which is the physiological modulator of colon mucosal cell maturation and may regulate intestinal homeostasis, not only via its effect on the proliferation and apoptosis levels, but also via the modulation of the equilibria and localization of various cell lines. This suppressive effect of butyrate on secretory and absorbing cells *in vivo* may play a significant role in the maintenance of intestinal homeostasis.

In the light of the above described state of technology, the goal of the present invention is to provide a solution facilitating the even delivery of a therapeutic dose of butyric acid or its pharmaceutically permissible salt, particularly sodium butyrate, into distant sections of the small and large intestines following oral administration in patients requiring it, particularly those suffering from diseases of the intestinal mucosa, such as inflammation, ulcers, or tumours. The possible administration of sodium butyrate as a coated, intraintestinal tablet, would result in the effects of sodium butyrate being limited to the site of tablet degradation. To date, the known methods of administering therapeutic sodium butyrate into distal portions of the human bowel made use of enemas. The disadvantage of the latter method is the necessity to perform frequent enemas, greatly reducing the patient's comfort.

A particular goal of present invention is to obtain an oral form of therapeutic preparation, particularly for the treatment and prophylaxis of acute and chronic bowel inflammation of varying ethiology and in the therapy of colorectal cancer in humans, where the form would facilitate the active release of sodium butyrate in more distal regions of the intestine, wherein it is preferential, that the active ingredient is released evenly and over the longest possible length of intestine.

The crux of the present invention is the application of sodium butyrate protected by a carefully selected triglyceride matrix, by itself or as a mixture of short-chain fatty acids, as a drug for the treatment of the bowel inflammation in humans. The drug is administered in an oral form, which facilitates the slow release of sodium butyrate in particular sections of the large and small intestines, or evenly throughout its length.

The goal of the present invention is a preparation containing sodium butyrate, characterized in that it contains: sodium butyrate protected in a triglyceride matrix at a concentration of 200 mg/g to 400 mg/g of preparation wherein the triglyceride matrix is up to 1 g, preferably the preparation additionaly comprises short-chain organic acids at 0 to 100 mg/g of preparation, wherein the matrix is characterized by acidity index below 2,0 mg KOH/g, saponification index from 200 to 210 KOH/ g and its liquefaction point is from 59,5 to 62°C, and the following percentage composition of carbohydrate chains of the fatty acids contained in the triglycerides: C12 app. 0,3%, C14 app. 1,5%, C16 from app. 52% up to app. 57%, C18 from app. 42% up to app. 47%, C20 and longer app. 1,2%..

Preferentially, the the triglyceride matrix with sodium butyrate is in the form of a microgranulate to be administered orally in powder form. Preferably is in the form of a capsule or tablet.

During research on favourable methods of administering sodium butyrate, it unexpectedly turned out that it is possible to design a preparation for use in humans which, when administered orally, will facilitate the controlled release of active sodium butyrate and other evaluated active substances in distal portions of the small intestine and in the colon, in a form facilitating both the administration and storage of the preparation. Triglyceride matrixes with sodium butyrate in the form of a microgranulate were designed to be administered *orally* as a powder or apportioned in hard caps, particularly cellulose ones, or as tablets. Research to date has shown the positive influence of butyric acid/sodium butyrate in the treatment of bowel inflammations and ulcerations and colorectal cancer, whereas a form which would facilitate the dosing of butyric acid/sodium butyrate intraintestinally easily and non-invasively in humans has not yet been proposed.

The addition of short chain fatty acids may enhance sodium butyrate activity, not altering the activity of the preparation.

It is preferential in the first distinct preparation according to the present invention, that:
- triglyceride matrices with sodium butyrate are apportioned in hard capsules, preferentially cellulose ones for administration *orally*.

The second distinct preparation containing sodium butyrate, according to the present invention, is characterized by the fact that it contains sodium butyrate protected in a triglyceride matrix at a concentration of 200 mg to 400 mg/tablet and/or short-chain organic acids at 0 to 100 mg/tablet and the triglyceride matrix to 1 g.

It is also advantageous, in the second distinct preparation according to the present invention, if sodium butyrate is used at a concentration of 300 mg/tablet or sodium butyrate is used at 300 mg/tablet with fumaric acid at 80 mg/tablet and the triglyceride matrices with sodium butyrate are in the form of tablets to be administered *orally.*

The application of a preparation or the first distinct preparation or the second distinct preparation in the manufacturing of a medicinal product for the treatment of intestinal inflammation and ulcers bowel inflammations, ulcerations and colorectal cancers, wherein the produced medicinal product is administered *orally* exclusively in humans in the form of a granulate, capsules or tablets.

Preferentially the medicinal product produced is administered to intestinal inflammation patients at 2 - 4 capsules a day, and if the medicinal product is administered during the treatment of colorectal cancer, it is given at 2 - 4 tablets twice a day or 4-8 capsules twice a day.

The next subject of the present invention is a matrix for the intra-intestinal release of medication, specific for a composition of natural triglycerides acidities of less than 2.0 mg KOH/g, saponification coefficients of 200 to 210 mg KOH/g, melting points of 59.5 to 62°C and the following percentage composition of various fatty acids with the following carbohydrate chain lengths contained in the triglycerides: C12 ca. 0.3%, C14 ca. 1.5%, C16 from about 52% to about 57%, C18 to about 42% to about 47%, C20 and about 1.2% of longer chains.

Preferentially, the matrix according to the present invention is obtained based on a natural plant oil fraction, particularly palm oil.

The next subject of the present invention is an application of the matrix according to the present finding in the preparation of an oral preparation of butyric acid or its pharmaceutically permissible salt in the treatment or prophylaxis of bowel inflammations and ulcerations and colon cancer. Preferentially, the preparation produced is used in the release of butyrate or its salt in more distal regions of the small intestine and in the colon. Equally preferentially, the preparation according to the present invention contains the matrix according to the present invention described above as the triglyceride matrix.

### Design of the composition of the matrix according to the present invention

A series of experiments were performed using triglyceride matrices of varying compositions in searching for a form of sodium butyrate to be administered orally, which would facilitate the introduction of sodium butyrate into the colon, with the simultaneous stimulation of small intestine cells.

The triglyceride matrix consists of a certain lipid fraction isolated from palm oil, where the proportions of the varying components have been experimentally determined.

An artificial intestine model was constructed for the purpose of evaluating the properties of the triglyceride matrix and comparing the functioning of particular fractions of saturated fatty acids.

A temperature of +37°C is maintained in this model, the medium is a phosphate-buffered saline - PBS, pH 7.2-7,.5 (adjusted using 5 M NaOH and 5 M HCl). A precisely determined amount of microgranulate is put into the system, as well as is an aqueous solution of porcine lipase (Cat. Nr L0382, Sigma-Aldrich) in such an amount so as to obtain an initial lipase activity of 400-600 U/ml measured spectrophotometrically using the Lipase-PS kit (nr. Kat. 805A-1KT, Trinity-Biotech, Ireland). Mixing occurs using forced pistoning at 3 rpm. The moment of introduction of the lipase constitutes time T₀ of the experiment. The degradation of the triglyceride matrix and by the same token of the release of sodium butyrate is measured by measuring the increase of sodium butyrate in the aqueous solution, which is the environment for the process.

From the time of passage of contents in the small intestine, it can be seen that the minimal time of release should be no less than 2 h.

In the experiments and comparisons commercially available palm oil derivatives including those made by Peter Cremer GmbH, Cremer Oleo were used.

The fractions as used in the experiments:

Fraction and standard:

| | |
|---|---|
| Liquefaction point | 56°C |

Percentage composition of carbohydrate chains (%):

| | |
|---|---|
| C12 | 1 |
| C14 | 1 |
| C16 | 59 |
| C18 | 37.5 |
| C20 and longer | 1.5 |

Fraction II standard:

| | |
|---|---|
| Liquefaction point | 65°C |

Percentage composition of carbohydrate chains (%):

| | |
|---|---|
| C16 | 6 |
| C18 | 92 |
| C18:1 | 1 |
| C18:2 | 1 |

Fraction III standard:

| | |
|---|---|
| Liquefaction point | 59°C |

Percentage composition of carbohydrate chains (%):

| | |
|---|---|
| C12 | 0,4 |
| C14 | 1,3 |
| C16 | 56,9 |
| C18 | 41 |
| C20 and longer | 0,4 |

Fraction IV standard:

| | |
|---|---|
| Liquefaction point | 60°C |

Percentage composition of carbohydrate chains (%):

| | |
|---|---|
| C12 | 1 |
| C14 | 1,3 |
| C16 | 30 |
| C18 | 66,7 |
| C20 and longer | 1 |

To achieve our technical goal, we composed compositions with different fractions of C14, C16, C18. The content of C12, C20 and higher organic acid residues were a result of the concentrations of these fractions in natural triglycerides used to compose the final product. As a result of the analysis of the effect of the matrix on the conditions inside the intestines, we eliminated highly acidic fractions of the contents, as they greatly influenced the pH of the system. During the experiment, it was unexpectedly shown that particular compositions of saturated fractions, C14, C16, C18 exert a good protective effect over sodium butyrate (its slow release) over time, throughout which, no less than 50% of the initial sodium butyrate should be released, following oral administration into a patient's bowel. It is equally important that this fraction also fulfilled the remaining requirement.

The preferential matrix composition is:

| | |
|---|---|
| Acidity index | below 2,0 mg KOH/g |
| Saponification index | 200-210 mg KOH/g |
| Liquefaction point | 59,5-62°C |

Percentage composition of carbohydrate chains (%):

| | |
|---|---|
| C12 | 03 |
| C14 | 1,5 |
| C16 | 52-57 |
| C18 | 42-47 |
| C20 and longer | 1,2 |

The liquefaction temperature was an important parameter, since it determines the longevity and technical parameters of the microgranulate which determine the storage and encapsulation processes. It was preferential to obtain a liquefaction temperature of no less than +60°C. This parameter is a product of the degree of saturation of the organic acids composing the triglycerides and carbon chain length.

The composition of the preferential fraction ensures the hardness and liquefaction temperature facilitate the easy production of microgranules with repeatable geometric parameters, sufficiently stable and dispersible that it was possible to pelletize bags of the product and to ship it considerable distances without loss of consistency.

Additionally, the preferential mixture of saturated triglyceride is characterised by a low acidity index high degree of saturation, a preferential liquefaction point and an ideal sodium butyrate release rate measured *in vitro* in the presence of lipase.

The results obtained were gathered in a table, where time means the period elapsed from T₀, the time of addition of the lipase, and the percentage value denotes the portion of matrix which undergoes no further transfer:

| Fraction/time | 30 min | 60 min | 90 min | 120 min | 150 min |
|---|---|---|---|---|---|
| Fraction and standard | 34% | 46% | 56% | 62% | 66% |
| Fraction II standard | 19% | 32% | 42% | 53% | 60% |
| Fraction III standard | 20% | 32% | 42% | 52% | 61% |
| Fraction IV standard | 24% | 38% | 46% | 52% | 59% |
| Experimental matrix | 18% | 30% | 38% | 45% | 49% |

Examination of the relative sodium butyrate release rate *in vitro* from the matrix in the model system made it possible to confirm the unexpected effect of the qualitative and quantitative composition of saturated triglycerides derived from palm oil, while simultaneously fulfilling all other technical and technological requirements.

Particular embodiments of the present invention have been presented in the examples below.

Example I. A mixture containing 300 mg of sodium butyrate with triglycerides from plants up to 1 g is made and then granules are formed, with diameters dependent on the final application: microgranules for administration in the form of a capsule and microgranules for spoon dosing.

The granulate is analysed chemically and then apportioned, into hard capsules, preferentially cellulose capsules, or into boxes.

The capsules are packed into PE containers using counters. The packaging is labelled, subjected to quality control, and then packed in cumulative packaging and palletized. Upon release from quality control, the packaging is directed to distribution storage.

Example II. A mixture containing 300 mg of sodium butyrate, 80 mg fumaric acid with triglycerides from plants up to 1 g is made as well as granules with diameters dependent on the final application are formed: microgranules for administration in the form of a capsule and microgranules for spoon dosing.

The granulate is analysed chemically and microbiologically and then apportioned, into hard capsules, preferentially cellulose capsules, or into boxes.

The capsules are packed into PE containers using counters. The packaging is labelled, subjected to quality control, and then packed in cumulative packaging and palletized. Upon release from quality control, the packaging is directed to distribution storage.

### Example III. (Reference Example)

A mixture is prepared consisting of sodium butyrate at 150 mg/capsule and 30% extract from *Yucca Schidigeri* at 2 mg/capsule with triglycerides from plants up to 0.5 g with diameters dependent on the final application: microgranules for administration in the form of a capsule and microgranules for spoon dosing.

The granulate is analysed chemically and microbiologically and then apportioned, into hard capsules, preferentially cellulose capsules, or into boxes.

The capsules are packed into PE containers using counters. The packaging is labelled, submitted to quality control, and then packed in collective packaging and palletized. Upon release from quality control, the packaging is directed to distribution storage.

### Example IV. (Reference Example)

A mixture is prepared consisting of sodium butyrate at 150 mg/capsule, fumaric acid at 40 mg/capsule and 30% extract from *Yucca Schidigeri* at 2 mg/capsule with triglycerides from plants up to 0.5 g with diameters dependent on the final application: microgranules for administration in the form of a capsule and microgranules for spoon dosing.

The granulate is analysed chemically and microbiologically and then apportioned, into hard capsules, preferentially cellulose capsules, or into boxes.

The capsules are packed into PE containers using counters. The packaging is labelled, submitted to quality control, and then packed in collective packaging and palletized. Upon release from quality control, the packaging is directed to distribution storage.

Example V. A mixture containing 300 mg/tablet of sodium butyrate with triglycerides from plants up to 1 g /tablet is made in the form of a granulate.

The granulate is analysed chemically and microbiologically and then packed into tablets. The tablets undergo physico-chemical analysis. The tablets are packed into PE containers or blisters using counters. The packaging is labelled, submitted to quality control, and then packed in collective packaging and palletized. Upon release from quality control, the packaging is directed to distribution storage.

Example VI. A mixture containing 300 mg/tablet of sodium butyrate, fumaric acid at 80 mg/tablet with triglycerides from plants up to 1 g /tablet is made in the form of a granulate. The granulate is analysed chemically and microbiologically and then packed into tablets. The tablets undergo physico-chemical analysis. The tablets are packed into PE containers or blisters using counters. The packaging is labelled, submitted to quality control, and then packed in collective packaging and palletized. Upon release from quality control, the packaging is directed to distribution storage.

### Example VII. (Reference Example)

A mixture is prepared consisting of sodium butyrate at 150 mg/tablet and 30% extract from *Yucca Schidigeri* at 4 mg/tablet with triglycerides from plants up to 1 g in the form of a granulate.

The granulate is analysed chemically and microbiologically and then packed into tablets. The tablets undergo physico-chemical analysis. The tablets are packed into PE containers or blisters using counters. The packaging is labelled, submitted to quality control, and then packed in collective packaging and palletized. Upon release from quality control, the packaging is directed to distribution storage.

Example VIII. A mixture is prepared consisting of sodium butyrate at 300 mg/tablet, fumaric acid at 80 mg/tablet and 30% extract from *Yucca Schidigeri* at 4 mg/tablet with triglycerides from plants up to 1 g in the form of a granulate.

The granulate is analysed chemically and microbiologically and then packed into tablets. The tablets undergo physico-chemical analysis. The tablets are packed into PE containers or blisters using counters. The packaging is labelled, submitted to quality control, and then packed in collective packaging and palletized. Upon release from quality control, the packaging is directed to distribution storage.

### Bibliography

1. Akira Andoh, Bamba T, Sasaki M. Physiological and anti-inflammatory roles of dietary fibber and butyrate in intestinal functions. J Parenter Enter Nutr. 1999; 23(5): 70-73.
2. Aviv-Green C, Polak-Charcon S, Madar Z, Schwartz B. Different molecular events account for butyrate-induced apoptosis in two human colon cancer cell lines. J Nutr.. 2002; 132 (7): 1812-18.
3. Aviv-Green C, Polak-Charcon S, Madar Z, Schwartz B. Apoptosis cascade proteins are regulated in vivo by high intracolonic butyrate concentration: coleration with colon cancer inhibition. Oncol Res. 2000; 12: 83-95.
4. Bartlett JG. Clinical practice. Antibiotic-associated diarrhoea. N Engl J Med. 2002; Jan 31;346(5):334-9.
5. Binder HJ, Mehta P. Short-chain fatty acids stimulate active sodium and chloride absorption in vitro in the rat distal colon. Gastroenterology 1989; 96: 989-996.
6. Böcker U, Nebe T, Herweck F, Holta L, Panja A, Jobin C, Rossol S, Sartor RB. Butyrate modulates intestinal epithelial cell-mediated neutrophil migration. Clin Exp Immunol. 2003; 131(1): 53-60
7. Chapman MAS, Grahn MF, Boyle ME, Hutton M, Rogers J, Williams NS. Butyrate oxidation is impaired in the colonic mucosa of sufferers of quiescent ulcerative colitis. Gut 1994; 35(1): 73-76.
8. Chapman MA. The role of the colonic flora in maintaining a healthy large bowel mucosa. Ann R Coll Surg Engl. 2001; 83: 75-80.
9. Charney AN, Micic L, Egnor RW. Nonionic diffusion of short-chain fatty acids across rat colon. Am J Physiol. 1998; 274: G518-524.
10. Chen CC, Walker WA. Probiotics and prebiotics: role and clinical disease states. Adv Pediatr. 2005; 52: 77-113.
11. Eggenberger JC, Farid A. Diversion colitis. Curr Treat Options Gastroenterol. 2001; 4(3): 253-259.
12. Firmansyah A, Penn D, Lebenthal E. Isolated colonocytes metabolism of glucose, glutamine, n-butyrate, β-hydroxybutyrate in malnutrition. Gastroenterology. 1989; 97: 622-629.
13. Galvez J, Rodrigez-Cabezas ME, Zarzuelo A. Effects of dietary fibber on inflammatory bowel disease. Mol Nutr Food Res. 2005; 49(6): 601-608.
14. Guillemot F, Colombel JF, Neut C, Verplanck N, Lecomte M, Romond C. Treatment of diversion colitis by short-chain fatty acids Dis Colon Rectum. 1991; 34: 861-864.
15. Guilloteau P, Romé V, Le Normand L, Savary G, Zabielski R. Is Na-butyrate a growth factor in the preruminant calf? Preliminary results. J Anim Feed Sci. 2004; 13, Suppl. 1: 393-396.
16. Hadjiagapiou C, Schmidt L, Dudeja PK, Layden TJ, Ramaswamy K. Mechanisms of butyrate transportin caco-2 cells: role of monocarboxylate transporter. Am J Physiol. 2000; 279: G775-780.
17. Han PD, Burke A, Baldassano RN, Rombaeu JL, Lichtenstein GR. Nutrition and inflammatory bowel disease. Gastroenterol Clin North Am. 1999; 28: 423-443.
18. Hassig CA, Tong JK, Schreiber SL. Derived-derived butyrate and the prevention of colon cancer. Chem Biol. 1997; 4: 783-789.
19. Holtug K, Rasmussen HS, Mortensen PB. An in vitro study of short-chain fatty acid concentrations, production and absorption in pig (Sus Scrofa) colon. Comp Biochem Physiol. 1992; 103A(1): 189-197.
20. Janne P, Carpenter Y, Willems G. Colonic mucosal atrophy induced by a liquid elemental diet in rats. Am J Dig Dis. 1977; 22: 808-812.
21. Jeppesen PB, Mortensen PB. Enhancing bowel adaptation in short bowel syndrome. Curr Gastroenterol Rep. 2002; 4: 338-347.
22. Kanauchi O, Iwanaga T, Mitsuyama K. Germinated baryle ford-stuff feeding. A novel neutraceutical therapeutic strategy for ulcerative colitis. Digestion. 2001; 63, Suppl. 1: 60-67.
23. Kanauchi O, Suga T, Tochihara M, Hibi T, Naganuma M, Homma T, Asakura H, Nakano H, Takahama K, Fujiyama Y, Andoh A, Shimoyama T, Hida N, Haruma K, Koga H, Mitsuyama K, Sata M, Fukuda M, Kojima A, Bamba T. Treatment of ulcerative colitis by feeding with germinated barley foodstuff: first report of a multicenter open control trial. J Gastroenterol. 2002; 37 (Suppl 14): 67-72.
24. Kolomecki K. Hamowanie funkcji metaloproteinaz - możliwości zastosowania klinicznego. Onkol Pol. 2000; 3: 163-167.
25. Koruda MJ, Rolandelli RH, Bliss DZ, Hastings J, Rombeau JL, Settle RG. Parenteral nutrition supplemented with short-chain fatty acids: Effect on the small -bowel mucosa in normal rats. Am J Cl Nutr. 1990; 51: 685- 689.
26. Kotunia A, Woliński J, Laubitz D, Jurkowska M, Rome V, Guilloteau P, Zabielski R. Effect of sodium butyrate on the small intestine development in neonatal piglets feed by artificial sow. J Physiol Pharmacol. 2004, 55, S2: 59-68.
27. Kripke SA, Fox AD, Berman JM, Settle RG, Rombeau JL. Stimulation of intestinal mucosa growth with intracolonic infusion of short-chain fatty acids. J Parenter Enter Nutr. 1989; 13: 109-116.
28. Lionetti P, Callegari ML, Ferrari S. Enteral nutrition and microflora in pediatric Crohn's disease. J Parenter Enteral Nutr. 2005: 29(Suppl 4): S173-175.
29. Liu Q, Shimoyama T, Suzuki K, Umeda T, Nakaji S, Sugawara K. Effect of sodium butyrate on reactive oxygen species generation by human neutriphils. Scand J Gastroenterol. 2001; 36: 744-750.
30. McIntyre A, Gibson PR, Young GP. Butyrate production from dietary fibber and protection against large bowel cancer in rat model. Gut. 1993; 34: 386-391.
31. Mitsuyama K, Saiki T, Kanauchi O. Treatment of ulcerative colitis with germinated baryle foodstuff feeding: a pilot study. Aliment Pharmacol Ther. 1998; 12: 1225-1230.
32. Mortensen FV, Hessov I, Brike H, Korsgaard N, Nielsen H. Microcirculatory and trophic effects of short-chain fatty acids in the human rectum after Hartmann's procedure. Br J Surg. 1991; 78: 1208-1211.
33. Pender S, Quenn JJ, Sanderson JR, MacDonald TT. Butyrate upregulates stromelysin-1 production by intestinal mesenchymal cells. Am J Physiol Gastrointest Liver Physiol. 2000; 279: G918-G924.
34. Rabbani M, Akbert J, Hamidur Rahman ASM, Mul Isal M, Nasirul Islam KM, Alam K. Short- chain fatty acids improve clinical, pathological, and microbiologic features of experimental Shigellosis. Journal of Infectious Disease. 1999; 179(2) :390-397.
35. Rabbani GH, Albert MJ, Rahman H, Chowdhury AK. Short-chain fatty acids inhibit fluid and electrolyte loss induced by cholera toxin in proximal colon of rabbit in vivo. Dig Dis Sci. 1999; 44: 1547-1553.
36. Ramakrishna BS, Venkataraman S, Srinivasan P, Dash P, Young GP, Binder HJ. Amylase-resistant starch plus oral rehydration solution for cholera. N Engl J Med. 2000; 342: 90-92.
37. Reodriger WEW. Role of anaerobic bacteria in the metabolic welfare of the colonic mucosa in man. Gut. 1980; 21: 793 - 798.
38. Ritzhaupt A, Antony Ellis, Ken B, Hosie and Soraya P. Shirazi-Beechey. The characterization of butyrate transport across pig and human colonic luminal membrane. J Physiol. 1998; 507(3): 819:830.
39. Roller M, Rechkemmer G, Watzl G. Prebiotic inulin enriched with oligofructose in combination with the prebiotics Lactobacillus rhamnosus and Biffidobacterium lactis modulates intestinal immune functions in rats. J Nutr. 2004; 134: 153-156.
40. Sakata T. Stimulatory effect of short chain fatty acids on epithelial cell proliferation in the rat intestine: A possible explanation for trophic effects of fermentable fibber, gut microbes and luminal trophic factors. Br J Nutr. 1987; 58: 95-103.
41. Scheppach W, Bartram P, Richter A, Richter F, Dusel G, Liepold H, Hofstetter G, Ruthlein J, Kasper H. Effect of short-chain fatty acids on the human colonic mucosa in vitro. J Parenter Enter Nutr. 1992; 16: 43-48.
42. Scheppach W, Christ SU, Bartram HP, Richter F, Kasper H. Effects of short-chain fatty acids on the inflamed colonic mucosa. Scand J Gastroenterol Suppl. 1997; 222: 53-57.
43. Schneider SM, Girard-Popau F, Filippi J. Effects of Sacharomyces boulardi on faecal short-chain fatty acids and microflora in patients on long-term total enteral nutrition. World J Gastroenterol. 2005; 11: 6165-6169.
44. Schneider SM, Le GP, Girard-Pipau F. Total artificial nutrition is associated with major changes in the fecal flora. Eur J Nutr. 2000; 39: 248-255.
45. Segain JP, Raingeard de la Bletierre D, Bourreille A. Butyrate inhibits inflammatory responses through NK-kappaB inhibition: implications for Crohn's disease. Gut. 2000; 47: 397-403.
46. Thompson JS, Quigley EM, Palmer JM, West WW, Adrian TE. Luminal short-chain fatty acidsn and post-resection intestinal adaptation. J Parenter Enteral Nutr. 1996; 20: 338-343.
47. Velázquez OC, Ledered HM, Rombeau JL. Butyrate and the colonocyte, implication for neoplasia. Dig Dis Sci. 1996; 41(4): 727-739.
48. Welters CF, Heineman E, Thunnissen FB, van den Bogaard AE, Soeters PB, Baeten CG. Effect of dietary inulin supplementation on inflammation of pouch mucosa in patients with an ileal pouch-anal anastomosis. Dis Colon Rectum. 2002; 45: 621-627.
49. Whitehead RH, Young GP, Bhathal PS. Effects of short chain fatty acids on new human colon carcinoma cell line (LIM 1215). Gut. 1986; 27: 1436-1457.
50. Wilson JR, Weiser M. Colonic cancer cell (HT-29) adhesion to laminin is altered by differentiation: Adhesion may involve galactosyl transferase. Exp Cell Res. 1992; 201: 330-334.
51. Young VB, Schmidt TM. Antibiotic-associated diarrhoea accompanied by large-scale alteration in the composition of fecal microbiota. J Clin Microbiol. 2004; 42: 1203-1206.
52. Zapolska-Downar D, Siennicka A, Kaczmarczyk M, Ko 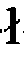 odziej B, Naruszewicz M. Butyrate inhibits cytokine-induced VCAM-1 and ICAM-1 expression In cultured endothelial cells: the role of NF-kappaB and PPAR alpha. J Nutr Biochem. 2004; 15: 220-228.

## Claims

1. A preparation containing sodium butyrate, **characterized in that** it contains: sodium butyrate protected in a triglyceride matrix at a concentration of 200 mg/g to 400 mg/g of preparation wherein the triglyceride matrix is up to 1 g, preferably the preparation additionally comprises short-chain organic acids at 0 to 100 mg/g of preparation, wherein the matrix is **characterized by** acidity index below 2,0 mg KOH/g, saponification index from 200 to 210 KOH/ g, a liquefaction point from 59,5 to 62°C, and the following percentage composition of carbohydrate chains of the fatty acids contained in the triglycerides: C12 app. 0,3%, C14 app. 1,5%, C16 from app. 52% up to app. 57%, C18 from app. 42% up to app. 47%, C20 and longer app. 1,2%.

2. A preparation according to claim 1, **characterized in that** the triglyceride matrix with sodium butyrate is in the form of a microgranulate to be administered orally in powder form.

3. A preparation according to claim 1, **characterized in that** it is in the form of a capsule or tablet.

4. A preparation according to Claim 3, **characterized in that** triglyceride matrices with sodium butyrate are apportioned in hard capsules, preferentially cellulose capsules, to be administered orally.

5. A preparation according to Claim 3, **characterized in that** it is in the form of tablet and sodium butyrate is used at a concentration of 300 mg/tablet and fumaric acid at 80 mg/tablet.

6. A preparation according to Claim 3, **characterized in that** the triglyceride matrices containing sodium butyrate are in the form of tablets to be administered orally.

7. A preparation according to claim 1 for use in the treatment or prophylaxis of intestinal inflammation and ulcers, as well as of colon cancer in humans.

8. A preparation for use according to Claim 7, **characterised in that** it is designed for oral administration in the form of a granulate, capsules or tablets.

9. A preparation for use according to Claim 7, **characterised in that** it is designed for administration in the treatment of intestinal inflammation at a rate of 2 - 4 capsules twice a day.

10. A preparation for use according to Claim 7, **characterised in that** it is designed for administration in the treatment of colon cancer at a rate of 2 - 4 tablets twice a day or 4-8 capsules twice a day.

## Patentansprüche

1. Zubereitung enthaltend Natriumbutyrat, **dadurch gekennzeichnet, dass** sie enthält: Natriumbutyrat, geschützt in einer Triglyceridmatrix in einer Konzentration von 200 mg/g bis 400 mg/g der Zubereitung, wobei die Triglyceridmatrix bis zu 1 g beträgt, vorzugsweise die Zubereitung zusätzlich kurzkettige organische Säuren von 0 bis 100 mg/g der Zubereitung enthält, wobei die Matrix **gekennzeichnet ist durch** einen Säureindex von unter 2,0 mg KOH/g, einen Verseifungsindex von 200 bis 210 KOH/g, einen Verflüssigungspunkt von 59,5 bis 62°C und die folgende prozentuale Zusammensetzung der Kohlenhydratketten der in den Triglyceriden enthaltenen Fettsäuren: C12 ca. 0,3%, C14 ca. 1,5%, C16 von ca. 52% bis zu ca. 57%, C18 von ca. 42% bis zu ca. 47%, C20 und länger ca. 1,2%.

2. Zubereitung nach Anspruch 1, **dadurch gekennzeichnet, dass** die Triglyceridmatrix mit Natriumbutyrat in Form eines oral in Pulverform zu verabreichenden Mikrogranulats vorliegt.

3. Zubereitung nach Anspruch 1, **dadurch gekennzeichnet, dass** sie in Form einer Kapsel oder Tablette vorliegt.

4. Zubereitung nach Anspruch 3, **dadurch gekennzeichnet, dass** Triglyceridmatrices mit Natriumbutyrat in Hartkapseln, vorzugsweise Cellulosekapseln portioniert sind, um oral verabreicht zu werden.

5. Zubereitung nach Anspruch 3, **dadurch gekennzeichnet, dass** sie in Form einer Tablette vorliegt und Natriumbutyrat in einer Konzentration von 300 mg/Tablette und Fumarsäure in einer Konzentration von 80 mg/Tablette verwendet wird.

6. Zubereitung nach Anspruch 3, **dadurch gekennzeichnet, dass** die Triglyceridmatrices, die Natriumbutyrat enthalten, in Form von Tabletten vorliegen, die oral verabreicht werden.

7. Zubereitung nach Anspruch 1 zur Verwendung bei der Behandlung oder Prophylaxe von Darmentzündungen und -geschwüren sowie von Dickdarmkrebs beim Menschen.

8. Zubereitung zur Verwendung nach Anspruch 7, **dadurch gekennzeichnet, dass** sie zur oralen Verabreichung in Form eines Granulats, Kapseln oder Tabletten ausgestaltet ist.

9. Zubereitung zur Verwendung nach Anspruch 7, **dadurch gekennzeichnet, dass** sie zur Verabreichung bei der Behandlung von Darmentzündungen mit einer Rate von 2 - 4 Kapseln zweimal täglich ausgestaltet ist.

10. Zubereitung zur Verwendung nach Anspruch 7, **dadurch gekennzeichnet, dass** sie zur Verabreichung bei der Behandlung von Dickdarmkrebs mit einer Rate von 2 - 4 Tabletten zweimal täglich oder 4 - 8 Kapseln zweimal täglich ausgestaltet ist.

## Revendications

1. Préparation contenant du butyrate de sodium, **caractérisée en ce qu'**elle contient : du butyrate de sodium protégé dans une matrice de triglycérides à une concentration de 200 mg/g à 400 mg/g de préparation dans laquelle la matrice de triglycérides va jusqu'à 1 g, de préférence la préparation comprend en plus des acides organiques à chaîne courte à 0 à 100 mg/g de préparation, dans laquelle la matrice est **caractérisée par** un indice d'acidité inférieur à 2,0 mg de KOH/g, un indice de saponification de 200 à 210 KOH/g, un point de liquéfaction de 59,5 à 62 °C, et la composition en pourcentage suivante de chaînes glucidiques des acides gras contenus dans les triglycérides : C12 app. 0,3 %, C14 app. 1,5 %, C16 d'app. 52 % à app. 57 %, C18 d'app. 42 % à app. 47 %, C20 et plus app. 1,2 %.

2. Préparation selon la revendication 1, **caractérisée en ce que** la matrice de triglycérides avec le butyrate de sodium est sous la forme d'un microgranulé à administrer par voie orale sous forme de poudre.

3. Préparation selon la revendication 1, **caractérisée en ce qu'**elle est sous la forme d'une capsule ou d'un comprimé.

4. Préparation selon la revendication 3, **caractérisée en ce que** des matrices de triglycérides avec du butyrate de sodium sont réparties dans des capsules dures, de préférence des capsules de cellulose, à administrer par voie orale.

5. Préparation selon la revendication 3, **caractérisée en ce qu'**elle est sous la forme d'un comprimé et le butyrate de sodium est utilisé à une concentration de 300 mg/comprimé et l'acide fumarique à 80 mg/comprimé.

6. Préparation selon la revendication 3, **caractérisée en ce que** les matrices de triglycérides contenant du butyrate de sodium sont sous la forme de comprimés à administrer par voie orale.

7. Préparation selon la revendication 1 pour une utilisation dans le traitement ou la prophylaxie d'une inflammation et d'ulcères intestinaux, ainsi que du cancer du côlon chez les humains.

8. Préparation pour une utilisation selon la revendication 7, **caractérisée en ce qu'**elle est conçue pour une administration orale sous la forme d'un granulé, de capsules ou de comprimés.

9. Préparation pour une utilisation selon la revendication 7, **caractérisée en ce qu'**elle est conçue pour une administration dans le traitement d'une inflammation intestinale à raison de 2 à 4 capsules deux fois par jour.

10. Préparation pour une utilisation selon la revendication 7, **caractérisée en ce qu'**elle est conçue pour une administration dans le traitement du cancer du côlon à raison de 2 à 4 comprimés deux fois par jour ou de 4 à 8 capsules deux fois par jour.
